Europäisches Patentamt

European Patent Office

Office européen des brevets

(11) Publication number: **0 590 403 A2**

# EUROPEAN PATENT APPLICATION

(21) Application number: 93114698.9

(22) Date of filing: 13.09.93

(51) Int. Cl.5: **A61K  35/26**, A61K 35/78,
    //(A61K35/78,35:26),
    (A61K35/78,33:06,31:51,31:525,
    31:44,31:68,31:355),(A61K35/26,33:06),(A61K35/78,
    35:26,33:06)

(30) Priority: 28.09.92 JP 283863/92

(43) Date of publication of application:
    06.04.94 Bulletin  94/14

(84) Designated Contracting States:
    BE DE FR GB LU NL

(71) Applicant: NISSEI MARINE IND. Co., Ltd.
    Kobayashi Bldg.,
    1-1-1, Hatagaya,
    Shibuya-ku
    Tokyo 151(JP)

(72) Inventor: Itakura, Hiroshige
    6-36, Hayamiya 2 chome
    Nerima-ku, Tokyo 176(JP)
    Inventor: Ikekawa, Nobuo
    21-5, Kichijoji Higashi-cho 2 chome
    Musashino-shi, Tokyo 180(JP)
    Inventor: Ikekawa, Tetsuro
    13-2, Makuhari Nishi 1 chome
    Mihama-ku

    Chiba-shi, Chiba-ken 281(JP)
    Inventor: Nakajima, Osamu
    43-20, Sakuragaoka 4 chome
    Tama-shi, Tokyo 206(JP)
    Inventor: Yamagami, Sueto
    26-10-605, Chuo 4 chome
    Nakano-ku, Tokyo 164(JP)
    Inventor: Tomonari, Masayuki
    9-16-G506, Shirakawa 4 chome
    Koto-ku, Tokyo 135(JP)
    Inventor: Ishihama, Yoshiaki
    3-15-203, Tobitakyu 1 chome
    Chofu-shi, Tokyo 182(JP)
    Inventor: Kihara, Toshio
    22-33-902, Higashi Gotanda 5 chome
    Shinagawa-ku, Tokyo 141(JP)

(74) Representative: Prüfer, Lutz H.
    PRÜFER & PARTNER,
    Patentanwälte,
    Harthauser Strasse 25d
    D-81545 München (DE)

(54) Compositions having analgesic action.

(57) The present invention provides compositions having an analgesic action, which contain an ingredient obtained from the thymus of a mammal as their primary and active component.

BACKGROUND OF THE INVENTION

1) Field of the Invention:

This invention relates to compositions which exhibit analgesic action in various diseases accompanied by pain and are useful in the field of foods and drugs.

2) Discussion of Related Art:

Many diseases are accompanied by pain, to a greater or lesser degree, from a transient and light pain to a chronic and intolerable sharp pain.

Complaints of arthralgia, neuralgia, pains in the low back and numbness of hands and legs increase as the age of patients advances since they are much or less involved in geriatric diseases. Accordingly, increase in the number of senior people in recent years in Japan has inevitably brought about a rapid increase in the number of patients who complain of pain.

However, today's developed medical science or medical treatment has still been unsuccessful in relieving the pain; inter alia, chronic pains involve many problems. It is only in recent dozen or so years that the medicinal and medical world has realized that chronic pains and acute pains constitute two different categories of pain. Pain treatments, therefore, are still staying in the stage of trial and error.

Of course, there are many analgesics which are commercially available and in use, and there are many methods proposed for relieving the pain, too.

However, their effects are not necessarily satisfactory, and in many cases, they involve adverse side effects. For instance, pains accompanied by cancer may be mentioned as a typical example of pain. In cases of patients in the terminal stage who have agonies with the persistently attacking severe pains, relief from the pain is given the highest priority by the medical service. In this connection, the World Health Organization (WHO) has recently taken up this subject, and has established a standard for the methods of relieving pain. Although it is said that 70 to 90% of the patients in the terminal stage should be relieved from the severe pain if they are treated by the methods proposed by the WHO, many patients do not enjoy the promised relief.

This is because the WHO methods involve adverse side effects and cause a serious constipation, and therefore, considerable manpower and time are required to cope with it. It is not only with this example, but many analgesics bring about various adverse side effects, more or less, such as renal disorders, liver disorders and so on.

As described above, it is the present situation that success in the development of drugs which have a sufficient analgesic effect with substantially no or very much reduced adverse side effects has not been yet in sight.

Under the above circumstances, the present inventors have conducted careful studies in an attempt to break the mentioned deadlock situation and to provide compositions having minimized adverse side effects and having much more excellent analgesic action than conventional analgesics, leading to completion of the present invention.

SUMMARY OF THE INVENTION

An object of the present invention is to provide a composition which comprises a tissue homogenate of thymus of a mammal as its primary active component.

The above and other objects, features and advantages of the present invention will become apparent from the following description.

DETAILED DESCRIPTION OF THE INVENTION AND PREFERRED EMBODIMENTS

The human body is provided with a function of suppressing or eliminating pains such as a sharp pain which discomfort a living body. Recent development in the life science has confirmed that this function is attributed to analgesic peptides such as endorphins, enkephalins and dinorphins, which have a function analogous to morphine. These analgesic peptides are present in the living body in the form of precursors, and these precursors are activated in response to the noxious stimuli or the like for expressing their function. It has also been revealed that the activation is greatly influenced by the factor whether or not the immune system is perfectly functioning.

In the meantime, the thymus which is the central organ which takes major part in the immune system of a living body comes to its maximum size at age 12 - 13 in case of human. Thereafter, it gradually diminishes until it becomes only a vestige. This is in agreement with the fact that the human immune function lowers as the age advances.

In the Oriental medical science, there is an idea called "horui" which means a supplementary. According to the idea of "horui", disorders in a certain organ of a human patient can be ameliorated by an intake (or supplementing - "ho") of the same organ ("rui") of another animal.

Based on this idea, the present inventors have proceeded with their thought and considered that the human immune system could be ameliorated by the intake of the thymus of a mammal, thereby accelerating the activation of analgesic peptides in the body for obtaining an analgesic effect.

The composition of the present invention contains the thymus of a mammal as its primary active component. It is possible to further contain calcium and a germ of wheat. Vehicles may also be included, as needed.

Examples of the thymus of a mammal useful in the present invention includes the thymus of a calf, a lamb or a young pig. From the viewpoint of the abundant supply, calf is the most suitable thymus source.

The starting material, thymus, taken out from the animal, is minced and mashed, and subsequently treated with hot water at 100°C for 20 to 30 minutes, followed by freeze-drying. The freeze-dried homogenate material obtained is crushed and sieved for pulverizing and provided for use. Since the thymus contains abundant and well-balanced amino acids and minerals, and further thymosin and other thymic factors or the like, it is known to enhance the stamina and to promote the function of immune system as mentioned above.

The composition according to the present invention may optionally contain other analgesic ingredients selected from the group consisting of coix seed extract, Asclepiadaceae extract, Acronichia pedunculata (L.) Miq. extract, Millettia reticulata Benth extract, cassia bark extract, safflower extract, calcium, members of the vitamin B group and vitamin E. In addition to these, the present composition may further contain one or more members selected from the group consisting of rehmannia root powder, pine needle extract, zanthoxylum fruit extract and licorice root extract.

The coix seed, zanthoxylum fruit, Asclepiadaceae, pine needles, licorice root, Acronichia pedunculata (L.) Miq., Millettia reticulata Benth, cassia bark, and safflower are provided in their dried form and cut into fine strips. They are simultaneously extracted with 30% ethanol, and the extract solution is centrifugally filtrated for removing foreign matters, followed by spray-drying to dryness. The thus obtained solid product is pulverized and sieved to prepare a dried extract powder.

The rehmannia root powder is prepared by first washing and selecting the collected root of rehmannia, then drying in the sun, pulverized into a powder of 200 to 100 meshes, and further drying and sterilizing at 100°C for 10 to 15 minutes. It has an appearance of powder tea.

Having regard to the calcium, thiamine nitrate (vitamin B1), riboflavine (vitamin B2), pyridoxine hydrochloride (vitamin B6), cyanocobalamin (vitamin B12) and alphatocopherol (vitamin E), they are commercially available from the suppliers concerned as a product which meets the quality standards established in the field.

Each of the above-mentioned ingredients according to the present invention is further described in detail along with its action:

[Group 1]

Coix Seed Extract: Contains fatty oil coioxol $C_8H_7O_3N$, sterol, amino acids, vitamin B1, etc. Oriental medicine schools typified by the traditional Chinese medicine and the modern Chinese medicine acknowledge the following actions: Promotes diuresis, anti-viral, analgesic, antispasmodic and eliminates the arthralgia due to wind-cold-wetness.

Especially effective for neuralgia.

Asclepiadaceae Extract: Contains essential oils, vitamins A and B, etc. Action: Nutritious to the liver and the kidney. Strengthens the muscle and bone system. Anti-inflammatory action. Diminishes edema. Analgesic. Useful in the medical prescription for rheumatoid arthritis and arthralgia.

Acronichia pedunculata (L.) Miq. Extract: Primarily contains essential oils. Action: Eliminates the painfulness by means of regulating vital energy. Activates blood circulation.

Millettia reticulata Benth Extract: Contains milletol $C_{29}H_{50}O_2$ and iron. Action: Analgesic action is obtained from the mechanism that the general circulation is ameliorated by promoting the blood circulation.

Also effective for atrophies in hands and legs, numbness and paralysis.

Cassia Bark Extract: Contains cinnamic aldehide $C_9H_{8O}$, cinnamyl ethyl acetate, etc. Action: Effective for pyretolysis, treatment for dredging the meridian passage, invigorates the stomach, antimicrobial, antiviral and antifungus. Especially analgesic against the headache due to twitching of the head blood vessel.

Safflower Extract: Contains carthamin $C_{21}H_{22}O_{11}$, saflor yellow $C_{24}H_{35}O_5$. Action: Improves blood circulation and regulates menstrual disorders. Effective for menstrual pain, pains due to angina pectoris, contusion, sprain, and bruise, internal bleeding and swelling.

Calcium: Some pains are caused from osteoporosis, which means brittle bones due to pores inside, as seen in some pain cases of low back. As a radical therapy, reinforcement of bones by the calcium intake and the promotion of calcium absorption is needed. Moreover, as a medical term "psychogenic pain" indicates, there is a close correlation between the heart/nerve system and the sensation of pain. Calcium is effective for obtaining an analgesic effect by regulating the nerve system, thereby mitigating the pain.

Thiamine Nitrate (vitamin B1): Effective for suppressing numbness of hands and legs and neuralgia.

Riboflavine (vitamin B2): Immunoactivating action, protecting action on the skin and mucosa, and anti-inflammatory action. Since inflammation, typified by arthritis, is one of major causes of pain, analgesic effects are expected by suppressing the inflammation.

Pyridoxine Hydrochloride (vitamin B6): Has a regulating action on the skin and mucosa as well as on the nerve function. Hyper-reaction of algesthecia nerves is expected to be suppressed.

Cyanocobalamin (vitamin B12): Has an action of relieving pains of peripheral nerves.

alpha-Tocopherol (vitamin E): Has an action of promoting blood circulation and suppressing production of peroxides. Disorder of blood circulation is one of the causes of pain. In this case, pain can be relieved by promoting the blood circulation. Moreover, since free radicals of peroxides or the like are considered to play a major role in the mechanism of onset and development of inflammation, it is expected that effects of preventing and mitigating the pain can be obtained by inhibiting the formation of peroxides, thereby suppressing inflammation.

[Group 2]

Rehmannia Root Powder: Contains sitosterol, D-mannitol, iridoi glycoside catalpol, etc. Action: Enriches blood, clears away heat and cools blood, treatment for tonic the kidney, cardiotonic, diuretic, and nutrition and stamina.

Zanthoxylum Fruit Extract: 2-4% Essential oils, alpha sanshol $C_{16}H_{27}ON$, sanshoamide $C_{16}H_{25}O_2N$, tannin, etc. Action: Invigorates the stomach and regulates functions of intestine. Detoxication and analgesic effect especially on neuralgia.

Pine Needle Extract: Essential oils are the major components. According to the Oriental medicine, it is acknowledged to have actions of ameliorating sleeplessness, analgesic to arthralgia and sedation.

Licorice Root Extract: Contains glycyrrhizin as a major component. Action: Invigorates the digestive function by vital energy, clears away heat and toxic materials, analgesic and sedation. In addition, it has a function of harmonizing the formulation as a whole.

It is preferred that the above-mentioned ingredients be blended in the following proportions:

[Group 1]

| | |
|---|---|
| Coix Seed Extract | 6 to 24% by weight |
| Asclepiadaceae Extract | 5 to 20% by weight |
| Acronichia pedunculata (L.) Miq. Extract | 2.5 to 10% by weight |
| Millettia reticulata Benth Extract | 5 to 20% by weight |
| Cassia Bark Extract | 2.5 to 10% by weight |
| Safflower Extract | 2.5 to 10% by weight |
| Calcium | 0.6 to 2.7% by weight |
| Thiamine Nitrate (VB1) | 1.7 to 6.8% by weight |
| Riboflavine (VB2) | 3.4 to 13.6% by weight |
| Pyridoxine Hydrochloride (VB6) | 3.4 to 13.6% by weight |
| Cyanocobalamin (VB12) | 0.00003 to 0.00012% by weight |
| alpha-Tocopherol (Vitamin E) | 1.4 to 5.6% by weight |

[Group 2]

| Rehmannia Root Powder | 4.7 to 19% by weight |
|---|---|
| Zanthoxylum Fruit Extract | 1.2 to 4.8% by weight |
| Pine Needle Extract | 4.0 to 16.0% by weight |
| Licorice Root Extract | 2.3 to 9.2% by weight |

The proportions are all based on the total weight of the composition.

EXAMPLES

The present invention will hereafter be described by way of Examples, which however, should not be construed as limiting the invention thereto.

Example 1:

21.4 wt.% of a freeze-dried powder of the calf thymus, 2.5 wt.% of calcium and 66.1 wt.% of reducing maltose were blended, kneaded to prepare tablets each weighing 350 mg. 6 to 12 tablets were administered to patients complaining of various kinds of pain. The results are shown in Table 1.

Table 1

Clinical Test Data on the Analgesic Effects in Diseases Accompanied by Pain:

Total number of cases: 72
Patients: age: 18-75, sex: male 35, female 37
Effects: Remarkably effective 16, Effective 41, Ratio of total effective cases 79.2%

| No. of cases and categories of effects | Classification | | | | | | | | | | | | Total |
|---|---|---|---|---|---|---|---|---|---|---|---|---|---|
| | Remarkably effective (Remarkably improved) | | | | Effective (Improved) | | | | No effect (slightly improved·unchanged) | | | | |
| Dose per day (tablets) → Diseases or symptoms | 6 | 9 | 12 | ST | 6 | 9 | 12 | ST | 6 | 9 | 12 | ST | Total |
| Rheumatoid arthritis | | 1 | | 1 | 1 | 1 | 1 | 3 | 3 | 1 | | 4 | 8 |
| Rheumatic arthritis | 1 | 1 | 2 | 4 | 3 | 2 | 10 | 15 | 2 | 1 | | 3 | 22 |
| Ischialgia | | | 2 | 2 | | 1 | 1 | 2 | 2 | | 1 | 3 | 7 |
| Trigeminal neuralgia | | 1 | 2 | 3 | | 2 | | 2 | | | | | 5 |
| Intercostal neuralgia | 1 | | | 1 | | | 1 | 1 | | | | | 2 |
| Ancle arthritis | | | | | 2 | | 1 | 3 | | | | | 3 |
| Low back pain | | | | | 1 | | 1 | 2 | | | | | 2 |
| Ancle sprain | 2 | | | 2 | 2 | 1 | | 3 | | | | | 5 |
| Gout | | | | | | 1 | | 1 | | | | | 1 |
| Nervous headache | | | 2 | 2 | 2 | | 1 | 3 | | | | | 5 |
| Angioneurotic headache | | | 1 | 1 | 3 | | | 3 | | | | | 4 |
| Left articulatio periarthritis | | | | | 1 | | | 1 | | | | | 1 |
| Cervicobrachial syndrome | | | | | 1 | | 1 | 2 | 1 | | | 1 | 3 |
| Soft tissue inflammation around hip joint | | | | | | | | | 1 | | 1 | 2 | 2 |
| Total | 4 | 3 | 9 | 16 | 16 | 8 | 17 | 41 | 9 | 2 | 2 | 13 | 70 |

Example 2:

21.4 wt.% of a freeze-dried powder of the lamb thymus, 2.5 wt.% of calcium and 66.1 wt.% of reducing maltose were blended, kneaded to prepare tablets each weighing 350 mg. 6 to 12 tablets were administered to patients complaining of various kinds of pain. The results are shown in Table 2.

Table 2

Clinical Test Data on the Analgesic Effects in Diseases Accompanied by Pain:

Total number of cases: 67
Patients:  age: 18-75, sex: male 31, female 36
Effects:  Remarkably effective 14, Effective 38, Ratio of total effective cases 77.6%

| No. of cases and categories of effects<br>Dose per day (tablets)<br>Diseases or symptoms | Classification | | | | | | | | | | | | |
|---|---|---|---|---|---|---|---|---|---|---|---|---|---|
| | Remarkably effective (Remarkably improved) | | | | Effective (Improved) | | | | No effect (slightly improved·unchanged) | | | | |
| | 6 | 9 | 12 | ST | 6 | 9 | 12 | ST | 6 | 9 | 12 | ST | Total |
| Rheumatoid arthritis | | 1 | | 1 | | 1 | 1 | 2 | 2 | 2 | | 4 | 7 |
| Rheumatic arthritis | | 1 | 1 | 2 | 2 | 7 | 4 | 13 | 1 | 1 | | 2 | 17 |
| Disk herniation | | | | | | | | | | 1 | | 1 | 1 |
| Ischialgia | | | 2 | 2 | | 1 | 1 | 2 | 2 | | 1 | 3 | 7 |
| Trigeminal neuralgia | | 1 | 2 | 3 | | 2 | | 2 | | | | | 5 |
| Intercostal neuralgia | 1 | | | 1 | | | 1 | 1 | | | | | 2 |
| Ancle arthritis | | | | | 1 | 1 | 1 | 3 | | | | | 3 |
| Low back pain | | | | | 1 | | 1 | 2 | | | | | 2 |
| Ancle sprain | 2 | | | 2 | 1 | 1 | | 2 | | | | | 4 |
| Gout | | | | | | 1 | | 1 | | | | | 1 |
| Nervous headache | | | 2 | 2 | 1 | | 1 | 2 | 1 | | | 1 | 5 |
| Angioneurotic headache | | | 1 | 1 | 1 | | 2 | 3 | | | | | 4 |
| Radiculoneuritis | | | | | | 1 | | 1 | | | | | 1 |

EP 0 590 403 A2

... Cont'd

| | | | | | | | | | | | | Total |
|---|---|---|---|---|---|---|---|---|---|---|---|---|---|
| Right articulatio cubri synovitis | | | | 1 | | 1 | | | | | | | 1 |
| Left articulatio periarthritics | | | | 1 | 1 | 2 | 1 | | | 1 | | 1 | 3 |
| Cervicobrachial syndrome | | | | | 1 | 1 | | 1 | | 1 | 1 | | 2 |
| Soft tissue inflammation around hip joint | | | | | 1 | | | | 1 | | 2 | | 2 |
| Total | 3 | 3 | 8 | 14 | 9 | 15 | 14 | 38 | 9 | 4 | 2 | 15 | 67 |

Example 3:

21.4 wt.% of a freeze-dried powder of the thymus of a young pig, 2.5 wt.% of calcium and 66.1 wt.% of reducing maltose were blended, kneaded to prepare tablets each weighing 350 mg. 6 to 12 tablets were

8

administered to patients complaining of various kinds of pain. The results are shown in Table 3.

Table 3

Clinical Test Data on the Analgesic Effects in Diseases Accompanied by Pain:

Total number of cases: 69
Patients: age: 18-75, sex: male 30, female 39
Effects: Remarkably effective 15, Effective 39, Ratio of total effective cases 78.3%

| Diseases or symptoms | Remarkably effective (Remarkably improved) | | | | Effective (Improved) | | | | No effect (slightly improved · unchanged) | | | | Total |
|---|---|---|---|---|---|---|---|---|---|---|---|---|---|
| Dose per day (tablets) | 6 | 9 | 12 | ST | 6 | 9 | 12 | ST | 6 | 9 | 12 | ST | |
| Rheumatoid arthritis | | | 1 | 1 | | | 2 | 2 | 2 | 1 | | 3 | 6 |
| Rheumatic arthritis | | 1 | 1 | 2 | 4 | 7 | 2 | 13 | 1 | | 1 | 2 | 17 |
| Ischialgia | | | 2 | 2 | | 1 | 1 | 2 | 1 | 1 | 1 | 3 | 7 |
| Trigeminal neuralgia | | 1 | 1 | 2 | | 2 | | 2 | | 1 | | 1 | 5 |
| Intercostal neuralgia | 1 | | | 1 | | | 1 | 1 | | | | | 2 |
| Ancle arthritis | 1 | | | 1 | 1 | 2 | 1 | 4 | 1 | | | 1 | 6 |
| Knee arthritis | | 1 | 1 | 2 | 1 | | | 1 | | | | | 3 |
| Low back pain | | | | | 1 | | 1 | 2 | | | | | 2 |
| Ancle sprain | 1 | 1 | | 2 | 1 | 1 | | 2 | | | | | 4 |
| Gout | | | | | | 1 | | 1 | | | | | 1 |
| Nervous headache | | 1 | | 1 | 1 | | 1 | 2 | 1 | | | 1 | 4 |
| Angioneurotic headache | | 1 | | 1 | 1 | | 1 | 2 | | | | | 3 |
| Right articulatio cubri synoritis | | | | | | | 1 | 1 | | | | | 1 |

... Cont'd

|  | | | | | | | | | | | | |
|---|---|---|---|---|---|---|---|---|---|---|---|---|
| Left articulatio periarthritis |  |  |  | 1 |  | 1 | 2 | 1 |  |  | 1 | 3 |
| Cervicobrachial syndrome |  |  |  | 1 |  |  | 1 | 1 |  |  | 1 | 2 |
| Soft tissue inflammation around hip joint |  |  |  | 1 |  |  |  | 1 |  | 1 | 2 | 2 |
| Obsolete dislocation of right hip joint |  |  |  |  | 1 |  | 1 |  |  |  |  | 1 |
| Total | 3 | 6 | 6 | 15 | 12 | 15 | 12 | 39 | 9 | 3 | 3 | 15 | 69 |

Example 4:

The following ingredients were blended and kneaded to prepare tablets each weighing 450 mg. They were administered to patients complaining of various kinds of pain in amounts of 6 to 12 tablets per day depending on the severeness of the pain. The results are shown in Table 4.

| | |
|---|---|
| Calf Thymus Powder | 19.0% by weight |
| Coix Seed Extract | 12.5% by weight or more |
| Asclepiadaceae Extract | 10.3% by weight |
| Acronichia pedunculata (L.) Miq. Extract | 4.6% by weight |
| Millettia reticulata Benth Extract | 10.9% by weight |
| Cassia Bark Extract | 4.6% by weight |
| Safflower Extract | 4.6% by weight |
| Calcium | 1.4% by weight |
| Thiamine Nitrate (VB1) | 3.4% by weight |
| Riboflavine (VB2) | 4.0% by weight |
| Pyridoxine Hydrochloride (VB6) | 4.0% by weight |
| Cyanocobalamin (VB12) | 0.00007% by weight |
| alpha-Tocopherol (Vitamin E) | 2.7% by weight |
| Rehmannia Root Powder | 6.2% by weight |
| Pine Needle Extract | 4.6% by weight |
| Zanthoxylum Fruit Extract | 2.5% by weight |
| Licorice Root Extract | 4.6% by weight |

Table 4

Clinical Test Data on the Analgesic Effects in Diseases Accompanied by Pain:

Total number of cases: 87
Patients:  age: 14-77, sex: male 48, female 39
Effects:  Remarkably effective 26, Effective 46, Ratio of total effective cases 82.8%

| No. of cases and categories of effects / Dose per day (tablets) / Diseases or symptoms | Classification | | | | | | | | | | | | Total |
|---|---|---|---|---|---|---|---|---|---|---|---|---|---|
| | Remarkably effective (Remarkably improved) | | | | Effective (Improved) | | | | No effect (slightly improved·unchanged) | | | | |
| | 6 | 9 | 12 | ST | 6 | 9 | 12 | ST | 6 | 9 | 12 | ST | Total |
| Rheumatoid arthritis | | | 1 | 1 | | 2 | 2 | 4 | 2 | 1 | | 3 | 8 |
| Rheumatic arthritis | 3 | 1 | 3 | 7 | 9 | 3 | 4 | 16 | 2 | 1 | | 3 | 26 |
| Ischialgia | | | 2 | 2 | 1 | 1 | | 2 | 2 | | 1 | 3 | 7 |
| Trigeminal neuralgia | | 1 | 2 | 3 | | 2 | | 2 | | 1 | | 1 | 6 |
| Intercostal neuralgia | | | 1 | 1 | | | 1 | 1 | | | | | 2 |
| Ancle arthritis | | | 1 | 1 | 1 | 1 | 1 | 3 | | | | | 4 |
| Maxilla arthritis | 1 | 1 | | 2 | | | | | | | | | 2 |
| Low back pain | | | 1 | 1 | 1 | | 2 | 3 | | 1 | | 1 | 5 |
| Ancle sprain | 2 | | | 2 | 1 | 2 | | 3 | | | | | 5 |
| Gout | | | | | | 1 | | 1 | | | | | 1 |
| Nervous headache | | 1 | 1 | 2 | 2 | | 1 | 3 | 1 | | | 1 | 6 |
| Angioneurotic headache | | 1 | | 1 | 1 | 1 | 2 | 4 | 1 | | | 1 | 6 |
| Herpes zoster | | | 1 | 1 | | | | | 1 | | | 1 | 2 |

EP 0 590 403 A2

... Cont'd

| | | | | | | | | | | | | | |
|---|---|---|---|---|---|---|---|---|---|---|---|---|---|
| Left articulatio periarthritis | | | | | 1 | 1 | | 2 | | | | | 2 |
| Cervicobrachial syndrome | | 2 | | 2 | 1 | | 1 | 2 | | 1 | | 1 | 5 |
| Total | 6 | 7 | 13 | 26 | 18 | 14 | 14 | 46 | 9 | 5 | 1 | 15 | 87 |

Example 5:

The following ingredients were blended and kneaded to prepare tablets each weighing 450 mg. The obtained tablets were administered to patients complaining of various kinds of pain in amounts of 6 to 12

13

tablets per day depending on the severeness of the pain, and their effects were checked. The results are shown in Table 5.

| Calf Thymus Powder | 20.4% by weight |
| Coix Seed Extract | 12.5% by weight or more |
| Asclepiadaceae Extract | 10.3% by weight |
| Acronichia pedunculata (L.) Miq. Extract | 6.8% by weight |
| Millettia reticulata Benth Extract | 10.9% by weight |
| Cassia Bark Extract | 6.8% by weight |
| Safflower Extract | 6.0% by weight |
| Calcium | 0.8% by weight |
| Thiamine Nitrate (VB1) | 3.4% by weight |
| Riboflavine (VB2) | 4.0% by weight |
| Pyridoxine Hydrochloride (VB6) | 4.0% by weight |
| Cyanocobalamin (VB12) | 0.00007% by weight |
| alpha-Tocopherol (Vitamin E) | 2.7% by weight |
| Rehmannia Root Powder | 4.9% by weight |
| Pine Needle Extract | 5.0% by weight |
| Zanthoxylum Fruit Extract | 1.5% by weight |
| Licorice Root Extract | 2.3% by weight |

Table 5

Clinical Test Data on the Analgesic Effects in Diseases Accompanied by Pain:

Total number of cases: 100
Patients:  age: 15-76, sex: male 57, female 43
Effects:  Remarkably effective 26, Effective 57, Ratio of total effective cases 83.0%

| No. of cases and categories of effects / Dose per day (tablets) / Disease or symptoms | Classification | | | | | | | | | | | | |
|---|---|---|---|---|---|---|---|---|---|---|---|---|---|
| | Remarkably effective(Remarkably improved) | | | | Effective (Improved) | | | | No effect (slightly improved·unchanged) | | | | |
| | 6 | 9 | 12 | ST | 6 | 9 | 12 | ST | 6 | 9 | 12 | ST | Total |
| Rheumatoid arthritis | 1 | | | 1 | 1 | | 1 | 2 | 1 | 2 | | 1 | 4 |
| Rheumatic arthritis | 3 | 1 | 5 | 9 | 12 | 1 | 5 | 18 | 2 | | | 2 | 29 |
| Disk herniation | 1 | | | 1 | 1 | | 2 | 3 | 3 | | | 3 | 7 |
| Ischialgia | | | 2 | 2 | 1 | 1 | | 2 | 2 | | 1 | 3 | 7 |
| Trigeminal neuralgia | | 1 | 2 | 3 | | 2 | | 2 | | | | | 5 |
| Intercostal neuralgia | 1 | | | 1 | | | 1 | 1 | | | | | 2 |
| Ancle arthritis | | | | | 2 | | 1 | 3 | | | | | 3 |
| Low back pain | | | | | 1 | | 1 | 2 | | | | | 2 |
| Pain in low back and thigh | | | | | 2 | | | 2 | | | | | 2 |
| Ancle sprain | 2 | | | 2 | 2 | 1 | | 3 | | | | | 5 |
| Sprain in the right chest | | | | | 1 | | | 1 | | | | | 1 |
| Sprain in the right chest fascia | | | | | 1 | | | 1 | | | | | 1 |
| Gout | | | | | | 1 | | 1 | | | | | 1 |

EP 0 590 403 A2

... Cont'd

| | 1 | 2 | 3 | 4 | 5 | 6 | 7 | 8 | 9 | 10 | 11 | Total |
|---|---|---|---|---|---|---|---|---|---|---|---|---|
| Nervous headache | 1 | | 2 | 2 | 2 | | 1 | 3 | | | | 5 |
| Angioneurotic headache | | | 1 | 1 | 3 | | | 3 | | | | 4 |
| Herpes zoster | | 1 | | 1 | | | | | | | | 1 |
| Shoulder pain | | | | | | | | | 1 | | 1 | 1 |
| Left articulatio periarthritics | | | | | 1 | | | 1 | | | | 1 |
| Cervicobrachial syndrome | | | | | 1 | | | 1 | | | | 1 |
| Spinal disease | | | | | 2 | | | 2 | 2 | | 2 | 4 |
| Right articulatio cubri synovitis | | | | | 2 | | | 2 | | | | 2 |
| Radiculoneuritis | | | | | 1 | | | 1 | | | | 1 |
| Chest pain | | | | | | | | | 1 | 1 | 2 | 2 |
| Hip joint | | | | | 1 | 1 | | 1 | 1 | | 1 | 2 |
| Aseptic necrosis in hip joint | 1 | | | | | | | 1 | 1 | | 1 | 3 |
| Metastatic cancer in left hip joint | | | | | | 1 | | | 1 | | 1 | 1 |
| Right humeral lateral epicondylitis | 1 | | | 1 | | | | | | | | 1 |
| Productive spinal inflammation | 1 | | | 1 | | | | | | | | 1 |
| Hypertrophic spinal inflammation | | | | | | | | 1 | | | | 1 |
| Total | 11 | 3 | 12 | 26 | 37 | 8 | 12 | 57 | 15 | 2 | 17 | 100 |

Example 6:

The following ingredients were blended and kneaded to prepare tablets each weighing 450 mg. The thus obtained tablets were administered to patients complaining of various kinds of pain in amounts of 6 to

16

12 tablets per day depending on the severeness of the pain, and their effects were checked. The results are shown in Table 6.

| | |
|---|---|
| Calf Thymus Powder | 21.8% by weight |
| Coix Seed Extract | 11.2% by weight or more |
| Asclepiadaceae Extract | 10.3% by weight |
| Acronichia pedunculata (L.) Miq. Extract | 4.6% by weight |
| Millettia reticulata Benth Extract | 9.5% by weight |
| Cassia Bark Extract | 4.6% by weight |
| Safflower Extract | 4.6% by weight |
| Calcium | 0.8% by weight |
| Thiamine Nitrate (VB1) | 3.4% by weight |
| Riboflavine (VB2) | 4.0% by weight |
| Pyridoxine Hydrochloride (VB6) | 4.0% by weight |
| Cyanocobalamin (VB12) | 0.00007% by weight |
| alpha-Tocopherol (Vitamin E) | 2.7% by weight |
| Rehmannia Root Powder | 4.9% by weight |
| Pine Needle Extract | 5.5% by weight |
| Zanthoxylum Fruit Extract | 2.4% by weight |
| Licorice Root Extract | 4.6% by weight |

Table 6

Clinical Test Data on the Analgesic Effects in Diseases Accompanied by Pain:

    Total number of cases: 126
    Patients:  age: 14-81, sex: male 61, female 65
    Effects:  Remarkably effective 39, Effective 66, Ratio of total effective cases 83.33%

| No. of cases and categories of effects — Dose per day (tablets) — Diseases or symptoms | Classification | | | | | | | | | | | | |
|---|---|---|---|---|---|---|---|---|---|---|---|---|---|
| | Remarkably effective (Remarkably improved) | | | | Effective (Improved) | | | | No effect (slightly improved·unchanged) | | | | |
| | 6 | 9 | 12 | ST | 6 | 9 | 12 | ST | 6 | 9 | 12 | ST | Total |
| Rheumatoid arthritis | | | 5 | 5 | 1 | 2 | 1 | 4 | 2 | 2 | | 4 | 13 |
| Rheumatic arthritis | 3 | 1 | 5 | 9 | 8 | 7 | 11 | 26 | 6 | 1 | | 7 | 42 |
| Disk herniation | 1 | | | 1 | 1 | | 2 | 3 | 3 | 1 | | 4 | 8 |
| Ischialgia | | | 2 | 2 | 1 | 1 | | 2 | 2 | | 1 | 3 | 7 |
| Trigeminal neuralgia | | 1 | 2 | 3 | | 2 | | 2 | | | | | 5 |
| Intercostal neuralgia | 1 | | | 1 | | | 1 | 1 | | | | | 2 |
| Ancle arthritis | | 1 | 1 | 2 | 2 | | 1 | 3 | | | | | 5 |
| Low back pain | | | | | 1 | | | 1 | | | | | 1 |
| Pain in low back and thigh | | | | | 2 | | | 2 | 1 | | | 1 | 3 |
| Ancle sprain | 2 | | | 2 | 2 | 1 | | 3 | 1 | | | 1 | 6 |
| Gout | | | | | | 2 | - 2 | | | | | | 2 |
| Nervous headache | | 3 | 2 | 5 | 2 | | 1 | 3 | | | | | 8 |

... Cont'd

EP 0 590 403 A2

| | | | | | | | | | | | | Total |
|---|---|---|---|---|---|---|---|---|---|---|---|---|
| Angioneurotic headache | | | | 3 | 3 | | | 3 | | | | 6 |
| Herpes zoster | | 1 | | 1 | | | | | | | | 1 |
| Shoulder pain | 1 | 1 | | 2 | | 1 | | 1 | | | | 3 |
| Articulatio periarthritics | | 1 | | 1 | 1 | 1 | | 2 | | | | 3 |
| Cervicobrachial syndrome | | | 1 | 1 | 1 | 1 | | 1 | | | | 2 |
| Articulatio cubri synovitis | | | | | 2 | | | 2 | | | | 2 |
| Radiculoneuritis | | | | | 1 | | | 1 | | | | 1 |
| Soft tissue inflammation around hip joint | | | | | 1 | | 1 | 2 | | | | 2 |
| Aseptic necrosis in hip joint | 1 | | | 1 | 1 | | | 1 | 1 | | 1 | 3 |
| Total | 9 | 10 | 20 | 39 | 30 | 17 | 19 | 66 | 16 | 4 | 21 | 126 |

Example 7:

As mentioned above, there are may drugs for relieving pains and ameliorating syndromes accompanied by pain. Among them, some drugs containing thiamine nitrate (vitamin B1) and other components, inter alia, vitamin B series can readily be purchased at drug stores or pharmacies, and they are intended for ameliorating neuralgia, muscle pains, articulate pains, pains in the low back and other chronic pains. They are so-called Occidental drugs and have symptomatic therapy-oriented characteristics.

When the patients have already taken vitamin B drugs and they are in hope of a continued use of them together with the drug of the present invention, they will have more free options concerning the selection of drugs, because the formulations of the invention, based on the Oriental medicinal idea of emphasizing radical treatment, can be used in combination with any Occidental drugs. Therefore, the inventors have investigated effects of such semi-Oriental drugs containing both herb drugs and Occidental drugs to see there is any difference. The result was that the semi-Oriental drugs seemed to exhibit slightly slower effects especially at the initial stage of administration, but as shown in Example 7, there was finally found no significant difference over the data obtained in Examples 4, 5 and 6.

Example 7 test was conducted as follows: The following ingredients were blended and kneaded to prepare tablets each weighing 380 mg. The thus obtained tablets were administered to patients complaining of various kinds of pain in amounts of 6 to 12 tablets per day depending on the severeness of the pain, and their effects were checked. The results are shown in Table 7.

| | |
|---|---|
| Calf Thymus Powder | 22.1% by weight |
| Coix Seed Extract | 13.5% by weight or more |
| Asclepiadaceae Extract | 11.2% by weight |
| Acronichia pedunculata (L.) Miq. Extract | 5.0% by weight |
| Millettia reticulata Benth Extract | 10.3% by weight |
| Cassia Bark Extract | 5.0% by weight |
| Safflower Extract | 5.0% by weight |
| Calcium | 1.5% by weight |
| Rehmannia Root Powder | 10.3% by weight |
| Pine Needle Extract | 8.5% by weight |
| Zanthoxylum Fruit Extract | 2.6% by weight |
| Licorice Root Extract | 5.0% by weight |

Table 7

Clinical Test Data on the Analgesic Effects in Diseases Accompanied by Pain:

Total number of cases: 85
Patients:  age: 16-77, sex: male 51, female 34
Effects:  Remarkably effective 20, Effective 48, Ratio of total effective cases 80.0%

| No. of cases and categories of effects<br><br>Dose per day (tablets)<br><br>Diseases or symptoms | Classification | | | | | | | | | | | | |
|---|---|---|---|---|---|---|---|---|---|---|---|---|---|
| | Remarkably effective(Remarkably improved) | | | | Effective (lmproved) | | | | No effect (slightly improved·unchanged) | | | | |
| | 6 | 9 | 12 | ST | 6 | 9 | 12 | ST | 6 | 9 | 12 | ST | Total |
| Rheumatoid arthritis | | 1 | 2 | 3 | 1 | 2 | 1 | 4 | 2 | 2 | | 4 | 11 |
| Rheumatic arthritis | | 1 | 3 | 4 | 3 | 6 | 11 | 20 | 4 | 2 | | 6 | 30 |
| Disk herniation | 1 | | | 1 | 1 | | 2 | 3 | 2 | 1 | | 3 | 7 |
| Ischialgia | | | 2 | 2 | 1 | 1 | | 2 | 2 | | 1 | 3 | 7 |
| Intercostal neuralgia | 1 | | | 1 | | | 1 | 1 | | | | | 2 |
| Ancle arthritis | | 1 | 1 | 2 | | 1 | 1 | 2 | | | | | 4 |
| Low back pain | | | | | 1 | | | 1 | | | | | 1 |
| Pain in low back and thigh | | | | | 2 | | | 2 | | | | | 2 |
| Gout | | | | | | 2 | | 2 | | 1 | | 1 | 3 |
| Nervous headache | | 1 | 2 | 3 | 2 | | 1 | 3 | | | | | 6 |
| Angioneurotic headache | | 1 | 1 | 2 | | 2 | | 2 | | | | | 4 |
| Shoulder pain | | 1 | | 1 | | 1 | | 1 | | | | | 2 |

EP 0 590 403 A2

| | | | | | | | | | | | | | Total |
|---|---|---|---|---|---|---|---|---|---|---|---|---|---|
| Articulatio periarthritics | | | | | | | 1 | | 1 | | | | 1 |
| Cervicobrachial syndrome | | | 1 | 1 | 1 | | | | 1 | | | | 2 |
| Articulatio cubri synovitis | | | | | | 1 | 1 | | 1 | | | | 1 |
| Soft tissue inflammation around hip joint | | | | | 1 | 1 | 2 | | | 6 | 1 | | 2 |
| Total | 2 | 6 | 12 | 20 | 13 | 17 | 18 | 48 | 10 | 6 | 1 | 17 | 85 |

## Example 8:

All of the ingredients concerned with the present invention are edible materials per se or vitamins which are popularly used, and therefore, possibility of their adverse side effects is out of question. For the sake of

22

assuredness, however, before starting the testings of the afore-mentioned Examples, sample tablets of Example 4 were administered to 65 volunteers who gave a consent to the trial, and each volunteer took 12 tablets per day over 14 days. As a result, any kinds of adverse side effects had never been observed, and instead, the following effects indicative of promotion of health were noted: 1) somehow feel better and vigorous; 2) feel light and nimble; and 3) feel fine and refreshed when awakened in the morning. The panel of volunteers consisted of the following people:

Age of his or her 80's: male 2, female 3,

Age of his or her 70's: male 9, female 13,

Age of his or her 60's: male 8, female 13,

Age of his or her 50's: male 5, female 7,

Age of his or her 40's: male 3, female 2,

[Toxicity Test]

Since the thymus is one of mammals' organs, and is served as a cuisine material, the thymus powder should never be toxic. For confirmation, however, a toxicity test in terms of LD50 was conducted using an animal model. Animals provided were 12 male ddy mice (average body weight: 23.9 g) and 12 female mice (average body weight: 21.6 g). Freeze-dried powder of calf thymus was dissolved in water and orally administered to the mice via a stomach probe. The mice were observed in a cage of ambient temperature of 23 ± 2°C and humidity of 50 ± 5%. The dose was 100 mg per mouse as calculated in terms of the weight of freeze-dried powder of the calf thymus.

After the administration of the thymus powder, the test animals were observed with respect to their general conditions. 15 Days after the administration, all the mice were slaughtered, and submitted to autopsy for visual observation.

Observation for 14 days revealed no death at all. Accordingly, the LD50 value is 4.0 g or more per 1 kg body weight of mouse, which is converted to 240 g or more in case of a human weighing 60 kg. This value indicates that the thymus powder is not toxic and absolutely safe. Moreover, no strange behaviors or no toxic symptoms were observed on the mice after the oral administration of freeze-dried powder of calf thymus.

As described above, the compositions according to the present invention exhibit analgesic actions in various diseases accompanied by pain, and are free from adverse side effects. Accordingly, the compositions are very useful for health foods, drugs and the like.

**Claims**

1. A composition having an analgesic action characterized by comprising, as its primary active ingredient, the thymus of a mammal.

2. A composition having an analgesic action characterized by comprising, as its primary active ingredient, a powder of the thymus of a mammal and further a member selected from the group consisting of coix seed extract, Asclepiadaceae extract, Acronichia pedunculata (L.) Miq. extract, Millettia reticulata Benth extract, cassia bark extract, safflower extract, calcium, members of vitamin B series, vitamin E and mixtures thereof.

3. A composition having an analgesic action which comprises, as its primary active ingredient, the thymus of a mammal, and further at least one member selected from each of the following groups 1 and 2:

   group 1: coix seed extract, Asclepiadaceae extract, Acronichia pedunculata (L.) Miq. extract, Millettia reticulata Benth extract, cassia bark extract, safflower extract, calcium, members of vitamin B series, vitamin E and mixtures thereof;

   group 2: rehmannia root powder, pine needle extract, zanthoxylum fruit extract, licorice root extract and mixtures thereof.

4. A composition having an analgesic action characterized by comprising the following ingredients:

| thymus powder of a mammal | 10 to 40% by weight |
|---|---|
| coix seed extract | 6 to 24% by weight |
| Asclepiadaceae extract | 5 to 20% by weight |
| Acronichia pedunculata (L.) Miq. Extract | 2.5 to 10% by weight |
| Millettia reticulata Benth Extract | 5 to 20% by weight |
| cassia bark extract | 2.5 to 10% by weight |
| safflower extract | 2.5 to 10% by weight |
| calcium | 0.6 to 2.7% by weight |
| thiamine nitrate (VB1) | 1.7 to 6.8% by weight |
| riboflavine (VB2) | 3.4 to 13.6% by weight |
| pyridoxine hydrochloride (VB6) | 3.4 to 13.6% by weight |
| cyanocobalamin (VB12) | 0.00003 to 0.00012% by weight |
| alpha-tocopherol (Vitamin E) | 1.4 to 5.6% by weight |
| rehmannia root powder | 4.7 to 19% by weight |
| pine needle extract | 4.0 to 16.0% by weight |
| zanthoxylum fruit extract | 1.2 to 4.8% by weight |
| licorice root extract | 2.3 to 9.2% by weight, |

wherein the proportions are all based on the total weight of the composition.

5. The composition according to any one of Claims 1 to 4, wherein the mammal is a calf.

6. The composition according to any one of Claims 1 to 4, wherein the mammal is a lamb.

7. The composition according to any one of Claims 1 to 4, wherein the mammal is a young pig.

8. The composition according to any one of Claims 1 to 3, wherein the thymus is in a powder form.